(19) Europäisches Patentamt
European Patent Office
Office européen des brevets

(11) EP 3 205 335 B1

(12) # EUROPEAN PATENT SPECIFICATION

(45) Date of publication and mention of the grant of the patent:
**20.01.2021 Bulletin 2021/03**

(21) Application number: **15849727.1**

(22) Date of filing: **11.09.2015**

(51) Int Cl.:
***A61K 8/06*** (2006.01) ***A61K 8/86*** (2006.01)
***A61K 8/31*** (2006.01) ***A01N 25/30*** (2006.01)
***A01N 25/06*** (2006.01)

(86) International application number:
**PCT/JP2015/075843**

(87) International publication number:
**WO 2016/056350 (14.04.2016 Gazette 2016/15)**

(54) **EMULSION COMPOSITION FOR AEROSOL PREPARATION, AND AEROSOL AGENT**

EMULSIONSZUSAMMENSETZUNG FÜR AEROSOLZUBEREITUNG SOWIE AEROSOLMITTEL

COMPOSITION D'ÉMULSION POUR PRÉPARATION D'AÉROSOL, ET AGENT D'AÉROSOL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.10.2014 JP 2014209392**

(43) Date of publication of application:
**16.08.2017 Bulletin 2017/33**

(73) Proprietors:
- **Kanagawa University**
  **Yokohama-shi, Kanagawa 221-8686 (JP)**
- **Koike Chemical Co., Ltd.**
  **Tokyo 130-0013 (JP)**

(72) Inventors:
- **TAJIMA, Kazuo**
  **Yokohama-shi**
  **Kanagawa 221-8686 (JP)**
- **IMAI, Yoko**
  **Yokohama-shi**
  **Kanagawa 221-8686 (JP)**
- **OMI, Hiroyuki**
  **Shibukawa-shi**
  **Gunma 379-1102 (JP)**
- **TAKADA, Kenta**
  **Shibukawa-shi**
  **Gunma 379-1102 (JP)**
- **YAMAMOTO, Kaoru**
  **Shibukawa-shi**
  **Gunma 379-1102 (JP)**
- **OOHIRA, Tetsuya**
  **Shibukawa-shi**
  **Gunma 379-1102 (JP)**
- **EIGI, Nagisa**
  **Shibukawa-shi**
  **Gunma 379-1102 (JP)**

(74) Representative: **Ström & Gulliksson AB**
**Box 5275**
**102 46 Stockholm (SE)**

(56) References cited:
**EP-A1- 1 029 528** **EP-A1- 2 359 801**
**EP-A2- 1 754 532** **JP-A- H06 329 532**
**JP-A- 2000 319 643** **JP-A- 2004 256 486**
**JP-A- 2006 239 666** **JP-A- 2008 031 098**
**JP-A- 2009 286 769** **JP-A- 2010 110 685**
**JP-A- 2013 194 000**

- **KUNIEDA H. ET AL.: "Phase behaviour of polyoxyethylene hydrogenated castor oil in oil/water system", COLLOIDS AND SURFACES A: PHYSIOCHEMICAL AND ENGINEERING ASPECTS, vol. 109, 1996, pages 209-216, XP029367259, ISSN: 0927-7757, DOI: 10.1016/0927-7757(95)03456-0**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

The present invention relates to an emulsion composition for aerosol preparation, and an aerosol agent.

### BACKGROUND ART

The aerosol agent has been used in various applications, for example, personal products such as cosmetics and pharmaceuticals; household articles such as aromatics and deodorants; and pesticides.
There has hitherto been used, as an emulsion composition to be used in a starting solution in an aerosol agent, those emulsified by a surfactant. For example, Patent Document 1 discloses an aerosol agent comprising a starting solution composed of an emulsion composition containing an oil phase, an aqueous phase, a surfactant, and a lower alcohol having 1 to 3 carbon atoms; and a propellant.
Patent Document 1: Japanese Patent No. 3399578

### DISCLOSURE OF THE INVENTION

#### Problems to be Solved by the Invention

An object of the present invention is to provide a novel aerosol agent having excellent emulsion stability of an emulsion composition, that replaces a conventional aerosol agent, and an emulsion composition for aerosol preparation.
Means for Solving the Problems
The present inventors have found that when using, as a starting solution of an aerosol agent, an emulsion composition emulsified by using vesicles formed of an amphipathic substance which spontaneously forms the vesicles, or particles of a condensation polymer having hydroxyl groups, an emulsified state of the emulsion composition in the aerosol agent is stabilized, leading to completion of the present invention. More specifically, the present invention provides the followings.

(1) An emulsion composition for aerosol preparation, comprising an oil phase; an aqueous phase; and vesicles formed of an amphipathic substance which spontaneously forms the vesicles, or particles of a condensation polymer having hydroxyl groups, wherein the oil phase includes a polymer compound having hydrophilic groups and hydrophobic groups.
(2) An aerosol agent comprising a starting solution composed of the emulsion composition according to (1) and a propellant.
(3) The aerosol agent according to (2) for application to a solid surface.

#### Effects of the Invention

According to the present invention, it is possible to provide a novel aerosol agent having excellent emulsion stability of an emulsion composition, that replaces a conventional aerosol agent, and an emulsion composition for aerosol preparation.

### BRIEF DESCRIPTION OF THE DRAWINGS

Fig. 1 is a drawing showing an image of an aerosol agent prepared in Preparation 4 of Aerosol Agent, which is observed by a microscope. Fig. 1A shows an image of an S-based W/O three-phase emulsion composition, which is observed by a microscope, and Fig. 1B shows an image of a composition containing neither a B-based stock solution nor an S-based stock solution prepared by using water in place of the B-based stock solution or the S-based stock solution in Table 14, which is observed by a microscope.

### PREFERRED MODE FOR CARRYING OUT THE INVENTION

Illustrative embodiments of the present invention will be described in detail below, but the present invention is not limited to the following embodiments at all and modifications can be appropriately made without departing from the scope of the present invention.

<Aerosol Agent>

The aerosol agent of the present invention comprises a starting solution composed of an emulsion composition including an oil phase, an aqueous phase, and vesicles formed of an amphipathic substance which spontaneously forms the vesicles, or particles of a condensation polymer having hydroxyl groups; and a propellant. Therefore, the aerosol agent of the present invention is excellent in emulsion stability of the emulsion composition. The aerosol agent of the present invention is also excellent in adhesion when it is sprayed to adhere to a solid surface. The aerosol agent of the present invention includes both of those stored in a container and those which are not stored into a container.

A surfactant has been used to emulsify an emulsion composition in a conventional aerosol agent. There has been known, as an emulsification mechanism which is different from that of a surfactant, a mechanism of a three-phase emulsification method. In case of emulsification by a surfactant, hydrophilic moieties of the surfactant are oriented towards the aqueous phase and hydrophobic moieties are oriented towards the oil phase to weaken the surface tension, thus completing emulsification. To the contrary, in case of three-phase emulsification, particles of a condensation polymer including vesicles formed of an amphipathic substance which spontaneously forms the vesicles, or particles of a condensation polymer having hydroxyl groups are used in place of the surfactant. The vesicles or particles of a condensation polymer exist at the interface between the oil phase and the aqueous phase, and adsorb to the oil phase by an intermolecular force to form a three-phase structure of aqueous phase-emulsified dispersant phase-oil phase, thus completing emulsification. As mentioned above, an emulsion composition obtained by using the surfactant has hitherto been used as the starting solution in the aerosol agent. As mentioned above, emulsification by the surfactant has an emulsification mechanism which is completely different from that of emulsification by three-phase emulsification, so that the occurrence an assumable situation is considered when using the emulsion composition by three-phase emulsification as the starting solution in the aerosol agent, thus failing to imagine what emulsion stability is exhibited. However, the present inventors have found that an emulsion composition by three-phase emulsification is usable as a starting solution of an aerosol agent, and also the aerosol agent using the emulsion composition by three-phase emulsification as the starting solution is excellent in emulsion stability of the emulsion composition.

In case of emulsification using a conventional surfactant, when oil or functional granules is/are emulsified and dispersed in water, there is a need to select the surfactant according to a HLB of the oil, and to perform emulsification and dispersion. Moreover, required HLB value of the oil varies depending on the case when a W/O type emulsion is produced and an O/W type emulsion is produced, so that there is a need to select the required HLB value of the oil according to each case. Further, when using the surfactant alone, sufficient emulsion stability (for example, thermal stability, temporal stability) is not obtained, so that there is a need to use a plurality of surfactants. However, since the aerosol agent of the present invention is excellent in emulsion stability of the emulsion composition by using the three-phase emulsification method, it is easy to stabilize an emulsified state regardless of the HLB value of the oil. Even if vesicles or particles of the condensation polymer are used alone, it is easy to stabilize the emulsified state. The surfactant has low biodegradability, thus causing foaming, so that it is not preferable from the viewpoint of environment. Meanwhile, according to the aerosol agent of the present invention, foaming does not occur and emulsification can be performed in a stable manner without using the surfactant.

The aerosol agent of the present invention exhibits high adhesion to a solid surface during spraying as compared with a conventional surfactant. The reason is considered that, in case of three-phase emulsification, vesicles or particles of a condensation polymer for emulsification are not single molecules but particles having symmetry, thus adhering to both of an oil droplet surface and a solid surface. Therefore, the aerosol agent of the present invention is preferably suited for application to a solid surface.

(Emulsion Composition)

The emulsion composition in the present invention includes an oil phase, an aqueous phase, and vesicles formed of an amphipathic substance which spontaneously forms the vesicles, or particles of a condensation polymer having hydroxyl groups, and constitutes a starting solution in an aerosol agent of the present invention.

The structure of the emulsion composition is not particularly limited, and may be either an O/W type emulsion structure or a W/O type emulsion structure.

Examples of the oil contained in the oil phase in the present invention include, but are not particularly limited to, hydrocarbon oil, ester oil, higher alcohol, higher fatty acid, oils and fats, silicone oil, and the like. Of these, hydrocarbon oil and ester oil are preferable. Particularly, the hydrocarbon oil has low polarity and it is difficult to stabilize an emulsified state in case of the W/O type emulsion. Meanwhile, regarding the aerosol composition of the present invention, it is easy to stabilize the hydrocarbon oil because of excellent emulsion stability of the emulsion composition. Therefore, it is preferred to use the hydrocarbon oil. These oils may be used alone, or two or more oils may be used in combination.

Examples of the hydrocarbon oil include, but are not particularly limited to, liquid paraffin, squalane, squalene, ceresin, and the like. Of these, liquid paraffin is preferable. These hydrocarbon oils may be used alone, or two or more

hydrocarbon oils may be used in combination.

Examples of the ester oil include, but are not particularly limited to, diisostearyl malate, isopropyl myristate, ethylhexyl palmitate, octyl palmitate, octyl isopalmitate, isononyl isononanoate, isotridecyl isononanoate, methylheptyl laurate, hexyl laurate, glyceryl tri(caprylate/caprate), triethylhexanoin, neopentyl glycol dicaprate, cetyl octanoate, isocetyl stearate, isopropyl isostearate, isodecyl oleate, glyceryl tri(2-ethylhexanoate), pentaerythrite tetra(2-ethylhexanoate), 2-ethylhexyl succinate, diethyl sebacate, and the like. Of these, diisostearyl malate and isopropyl myristate are preferable since it is easy to be emulsified in a stable manner. These ester oils may be used alone, or two or more ester oils may be used in combination.

Examples of the higher alcohol include, but are not particularly limited to, isostearyl alcohol, octyldodecanol, oleyl alcohol, and the like. These higher alcohols may be used alone, or two or more higher alcohols may be used in combination.

Examples of the higher fatty acid include, but are not particularly limited to, isostearic acid, oleic acid, and the like. These higher fatty acids may be used alone, or two or more higher fatty acids may be used in combination.

Examples of oils and fats include, but are not particularly limited to, jojoba oil, olive oil, camellia oil, corn oil, mink oil, avocado oil, sasanqua oil, castor oil, safflower oil, sunflower oil, almond oil, rapeseed oil, sesame oil, soybean oil, and the like. These oils and fats may be used alone, or two or more oils and fats may be used in combination.

Examples of the silicone oil include, but are not particularly limited to, methylpolysiloxane, dimethylpolysiloxane, methylphenylpolysiloxane, methylhydrogenpolysiloxane, octamethylcyclotetrasiloxane, decamethylcyclopentasiloxane, dodecamethylcyclohexasiloxane, and the like. These silicone oils may be used alone, or two or more silicone oils may be used in combination.

In the present invention, in case of the W/O type emulsion, an dielectric constant of the oil phase is not particularly limited as long as the dielectric constant is 2.1 or more, and the dielectric constant is preferably 11 or less, more preferably 3.0 to 7.5, and still more preferably 3.1 to 4.0. The dielectric constant of the oil phase is measured by a liquid dielectric constant meter, Model 871 (manufactured by Nihon Rufuto Co., Ltd.). In case of the O/W type emulsion, there is no particular limitation on the dielectric constant of the oil phase.

There is no particular limitation on the content of the oil relative to the total mass of the emulsion composition in the present invention, and it is possible to appropriately select according to the form and object of the emulsion composition. For example, in case of the O/W type emulsion structure, the content of the oil is preferably 70% by mass or less, and more preferably 50% by mass or less, because it is easy to spray from the viewpoint of fluidity. For example, in case of the W/O type emulsion structure, the content of the oil is preferably 25% by mass or more, more preferably 50 to 95% by mass, and still more preferably 70 to 90% by mass.

Vesicles or particles of the condensation polymer in the present invention are significantly excellent in emulsification performances. Therefore, the amount of water in the emulsion composition can be widely selected from a range of 0.1 to 95% by mass, and can be appropriately set according to the form of the emulsion composition and the amount of the oil phase. For example, in case of the O/W type emulsion structure, the amount of water is preferably 30% by mass or more, and more preferably 50% by mass or more. For example, in case of the W/O type emulsion structure, the amount of water is preferably 75% by mass or less, more preferably 5 to 50% by mass, and still more preferably 10 to 30% by mass.

In the present invention, surfactants and other emulsifiers may be included or not in the aspect to be used, in addition to vesicles or particles of the condensation polymer. The content of the surfactant in the emulsion composition is not particularly limited and can be set, for example, within a range of 10% by mass or less (5% by mass or less, 1% by mass or less, 0.1% by mass or less, 0.05% by mass or less, 0.01% by mass or less, etc.) relative to the total mass of the emulsion composition.

Vesicles in the present invention are formed of an amphipathic substance which spontaneously forms vesicles. It is possible to employ, as the amphipathic substance which forms vesicles, derivatives of polyoxyethylene hardened castor oil represented by the general formula 1 mentioned below, or dialkylammonium derivatives, trialkylammonium derivatives, tetraalkylammonium derivatives, dialkenylammonium derivatives, trialkenylammonium derivatives represented by the general formula 2 mentioned below or derivatives of halogen salts of tetraalkenylammonium derivatives.

General Formula 1

[Chem. 1]

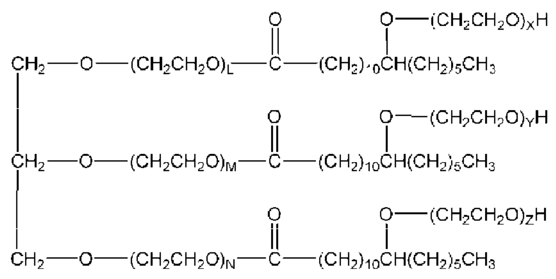

E=L+M+N+X+Y+Z

In the formula, E as an average addition molar number of ethylene oxide is 3 to 100. Excessive increase in E leads to restriction of types of a good solvent which dissolves the amphipathic substance, so that the degree of freedom in the production of hydrophilic nanoparticles decreases. The upper limit of E is preferably 50, and more preferably 40, while the lower limit of E is preferably 5.

General Formula 2

[Chem. 2]

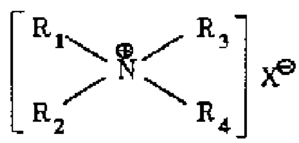

In the formula, R1 and R2 each independently is an alkyl or alkenyl group having 8 to 22 carbon atoms, R3 and R4 each independently is hydrogen or an alkyl group having 1 to 4 carbon atoms, and X is F, Cl, Br, I, or $CH_3COO$.

Alternatively, it is possible to employ phospholipid, phospholipid derivatives, or the like. It is possible to use, as the phospholipid, DLPC having a carbon chain length of 12 (1,2-Dilauroyl-sn-glycero-3-phospho-rac-1-choline), DMPC having a carbon chain length of 14 (1,2-Dimyristoyl-sn-glycero-3-phospho-rac-1-choline), or DPPC having a carbon chain length of 16 (1,2-Dipalmitoyl-sn-glycero-3-phospho-rac-1-choline) among members represented by the general formula 3 mentioned below.

General Formula 3

[Chem. 3]

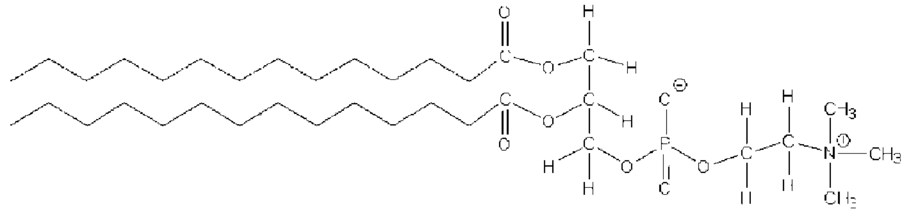

It is also possible to employ Na or $NH_4$ salts of DLPG having a carbon chain length of 12 (1,2-Dilauroyl-sn-glycero-3-phospho-rac-1-glycerol), Na or $NH_4$ salts of DMPG having a carbon chain length of 14 (1,2-Dimyristoyl-sn-glycero-3-phospho-rac-1-glycerol), or Na or $NH_4$ salts of DPPG having a carbon chain length of 16 (1,2-Dipalmitoyl-sn-glycero-3-phospho-rac-1-glycerol) among members represented by the general formula 4 mentioned below.

General Formula 4

[Chem. 4]

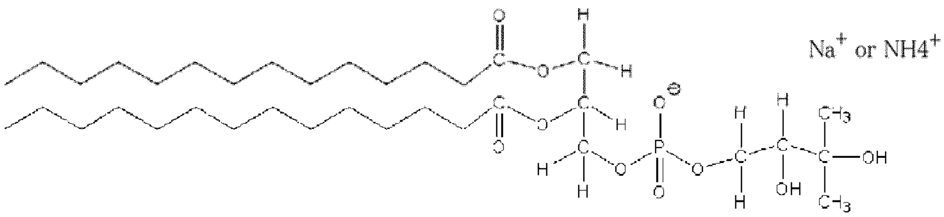

The amphipathic substance is preferably polyoxyethylene hardened castor oil, sodium dilauramidoglutamide lysine, or polyglycerin fatty acid ester. These amphipathic substances may be used alone, or two or more amphipathic substances may be used in combination. Particularly, when using a compressed gas $CO_2$ as a propellant, it is difficult to stabilize an emulsified state. However, use of polyglycerin fatty acid ester makes it easy to stabilize an emulsified state. From this point of view, it is preferred to use polyglycerin fatty acid ester.

The condensation polymer having hydroxyl groups is not particularly limited and may be either a natural polymer or a synthetic polymer, and may be appropriately selected according to purposes. The condensation polymer is preferably a natural polymer in view of the fact that it is excellent in safety and is commonly inexpensive, and more preferably a sugar polymer mentioned below since because of its excellent emulsified function. Particles include both of those in which condensation polymers are converted into single particles, or those in which these single particles are connected together, but do not include an aggregate (having a network structure) before conversion into single particles. These condensation polymers may be used alone, or two or more condensation polymers may be used in combination.

The sugar polymer is a polymer having a glucoside structure, such as cellulose or starch. Examples thereof include those produced by microorganisms using, as a constituent element, some saccharides selected from among monosaccharides such as ribose, xylose, rhamnose, fucose, glucose, mannose, glucuronic acid, and gluconic acid; natural polymers such as xanthan gum, gum arabic, guar gum, karaya gum, carrageenan, pectin, fucoidan, quince seed gum, gum tragacanth, locust bean gum, galactomannan, curdlan, gellan gum, Fucogel, casein, gelatin, starch, collagen, and Tremella fuciformis polysaccharide; semisynthetic polymers such as methyl cellulose, ethyl cellulose, methylhydroxypropyl cellulose, carboxymethyl cellulose, hydroxymethyl cellulose, hydroxypropyl cellulose, hydroxypropylmethyl cellulose, hydroxyethyl cellulose, stearoxyhydroxypropylmethyl cellulose, sodium carboxymethyl cellulose, propylene glycol alginate, cellulose crystal, starch-sodium acrylate graft polymer, and hydrophobized hydroxypropylmethyl cellulose; and synthetic polymers such as polyvinyl alcohol, polyvinylpyrrolidone, carboxyvinyl polymer, polyacrylate, and polyethylene oxide. These sugar polymers may be used alone, or two or more sugar polymers may be used in combination.

It is preferred to use, as sugar polymer particles, polysaccharide mixture such as agar, hydroxyethyl cellulose, stearoxyhydroxypropylmethyl cellulose, carboxymethyl cellulose, carboxymethyl cellulose, guar gum, or salts thereof. Particularly, when using dimethyl ether which is liquefied gas as a propellant, it is difficult to stabilize an emulsified state. However, use of stearoxyhydroxypropylmethyl cellulose makes it easy to stabilize an emulsified state. From this point of view, it is preferred to use stearoxyhydroxypropylmethyl cellulose.

There is no particular limitation on an average particle diameter of an inner phase of the emulsion composition in the present invention, and the average particle diameter may be, for example, 0.001 $\mu$m or more. Since the emulsion composition in the present invention is obtained by three-phase emulsification, when compared with conventional emulsification using a surfactant, it is possible to set the magnitude of the average particle diameter within a wide range. The average particle diameter of the oil phase may be appropriately set according to objects and applications. For example, when the aerosol agent is used for the purpose of adhesion, the average particle diameter of the inner phase is preferably 500 $\mu$m or less, more preferably 250 $\mu$m or less, and still more preferably 100 $\mu$m or less. The average particle diameter of the inner phase is measured by a particle size distribution analyzer in concentrated solutions FPAR-1000 (manufactured by Otsuka Electronics Co., Ltd.).

In vesicles or condensation polymer particles of the present invention, the average particle diameter is about 8 nm to 800 nm before formation of an emulsion, but the average particle diameter is about 8 nm to 500 nm in the O/W type emulsion structure. These preparation methods are omitted since they are conventionally known as disclosed in

JP 3855203 B1.

The amount of vesicles or condensation polymer particles may be appropriately set according to the amount of the oil phase and is not particularly limited, and may be 0.0001 to 5% by mass in total. Whereby, it is possible to satisfactorily maintain the emulsified state before use of the aerosol agent. Unlike the conventional surfactant, vesicles or condensation polymer particles have excellent emulsification properties, so that they can maintain an emulsified state even in a small amount such as 5% by mass or less (specifically, 4% by mass or less, 3% by mass or less, 2% by mass or less, 1.0% by mass or less, 0.75% by mass or less). Any of the amounts mentioned above is a solid content.

The emulsion composition in the present invention may include any known components included in the emulsion composition in a conventional aerosol agent. For example, when a polymer compound having hydrophilic groups and hydrophobic groups is included in an oil phase, hydrophilic groups are oriented towards the aqueous phase and hydrophobic groups are oriented towards the oil phase, leading to excellent stability of the emulsion composition in the aerosol agent. Therefore, it is preferred to include the polymer compound having hydrophilic groups and hydrophobic groups in the oil phase. Examples of the polymer compound having hydrophilic groups and hydrophobic groups include, but are not limited to, a saccharide having a fatty acid group, such as dextrin having a fatty acid group or inulin having a fatty acid group; and a polymer including acrylic acid or methacrylic acid, such as an acrylic acid-alkyl (meth)acrylate copolymer. Of these, dextrin having a fatty acid group and an acrylic acid-alkyl (meth)acrylate copolymer are preferable. There is no particular limitation on the number of carbon atoms of dextrin having a fatty acid group, and the number may be, for example, 1 to 30, preferably 4 to 28, more preferably 8 to 24, and still more preferably 10 to 22. It is most preferred to use dextrin palmitate or dextrin myristate.

There is no particular limitation on the content of the polymer compound having hydrophilic groups and hydrophobic groups in the emulsion composition, and the content may be, for example, 0.01 to 20% by mass. High effect of stabilizing the emulsion composition is exerted even when using a small amount of the polymer compound. Too large amount leads to an excessive increase in viscosity of the emulsion composition, so that the thus obtained emulsion composition is not suited for use in the aerosol agent. In view of such balance, the content of the polymer compound having hydrophilic groups and hydrophobic groups in the emulsion composition is preferably 5% by mass or less, more preferably 3% by mass or less, and still more preferably 1% by mass or less, relative to the total mass of the emulsion composition.

There is no particular limitation on the content of the oil in the entire oil phase in the present invention, and the content may be, for example, 10% by mass or more (20% by mass or more, 30% by mass or more, 40% by mass or more, 50% by mass or more, 60% by mass or more, 70% by mass or more, 80% by mass or more, 90% by mass or more, 95% by mass or more, or the like) and the oil phase may be composed only of the oil.

As mentioned above, there is no particular limitation on the viscosity of the emulsion composition in the present invention, and the viscosity can be appropriately set according to types of a propellant and applications of an aerosol agent.

The emulsion composition in the present invention may include, in addition to components other than those mentioned above, perfumes, pH adjustors, preservatives, thickeners, colorants, antioxidants, and the like. However, additives mentioned above are not limited thereto and may not be included. According to purposes, the above-mentioned polymer compound having hydrophilic groups and hydrophobic groups may be used as the thickener.

The emulsion composition can be prepared by mixing a dispersion including vesicles or condensation polymer particles with an oily component to form an O/W type or W/O type emulsion. A water-soluble optional component may be added before emulsification, or may be added after emulsification in case of the O/W type.

(Propellant)

There is no particular limitation on the propellant, and it is possible to use a compressed gas, a liquefied gas, or a mixed gas thereof.

There is no particular limitation on the compressed gas and, for example, $N_2$, $CO_2$, $N_2O$, and the like may be used. Alternatively, it is possible to use a mixture of a plurality of gasses, such as air. When using $CO_2$, it becomes difficult to stabilize an emulsified state of the emulsion composition. The aerosol agent of the present invention is excellent in emulsion stability of the emulsion composition, so that the emulsified state is stable even when using $CO_2$. Therefore, it is also possible to use $CO_2$ as the compressed gas.

There is no particular limitation on the pressure of the compressed gas. As defined in High Pressure Gas Safety Law, it is possible to adjust the pressure to less than 1 MPa at 35°C and to less than 1 MPa at normal temperature

Examples of the liquefied gas include, but are not limited to, butane, propane, dimethyl ether, fluorocarbon, liquefied petroleum gas, and the like. These liquefied gases may be used alone, or two or more liquefied gases may be used in combination. When using dimethyl ether among these liquefied gases, it becomes difficult to stabilize an emulsified state of the emulsion composition. The aerosol agent of the present invention is excellent in emulsion stability of the emulsion composition, so that the emulsified state is stable even when using dimethyl ether.

The content of the liquefied gas is not particularly limited and may be, for example, 1 to 90% by mass relative

to the total mass of a liquefied gas and a starting solution. When the amount of the liquefied gas is increased, it becomes difficult to stabilize the emulsified state. The aerosol agent of the present invention is excellent in emulsion stability of the emulsion composition, so that it is easy to stabilize the emulsified state even when increasing the amount of the liquefied gas. When the amount of the liquefied gas is excessively increased, it becomes impossible to stabilize the emulsified state. Therefore, the upper limit of the liquefied gas is preferably 95% by mass or less, more preferably 90% by mass or less, and still more preferably 85% by mass or less, relative to the total mass of a liquefied gas and a starting solution.

(Method for Producing Aerosol Agent)

An aerosol agent in the present invention can be produced according to a conventional method. For example, the aerosol agent can be produced by adding a starting solution composed of the thus prepared emulsion composition to a container, and filling the container with a propellant.

There is no particular limitation on the container which is filled with the aerosol agent, and it is possible to use those which can be used as a container of a conventionally known aerosol agent. For example, it is possible to use materials such as metal, resin, and glass. If necessary, the container may be equipped with those with which the container of a conventionally known aerosol agent is equipped, such as a valve, an actuator, a cap, and the like.

(Applications)

There is no particular limitation on applications of the aerosol agent of the present invention, and the aerosol agent can be used, for example, in personal products (cosmetics, quasi drugs, pharmaceuticals, etc.), household articles (aromatics, deodorants, waterproof agents, water repellent agents, laundry starches, herbicides, moth balls, fire extinguishers, germicides, cleaners), pesticides (space pesticides, cockroach insecticides, acaricides, etc.), automotive products (antifogging agents, thawing agents, etc.), industrial products (coating materials, adhesives, rust preventives, etc.), pet supplies, sporting goods, foods, and the like. As mentioned above, the aerosol agent of the present invention is excellent in adhesion to a solid surface. Thus, the aerosol agent of the present invention is preferably used for application to a solid surface. Specifically, the aerosol agent is preferably used in cosmetics, quasi drugs, waterproof agents, water repellent agents, laundry starches, herbicides, germicides, cleaners, cockroach insecticides, acaricides, antifogging agents, thawing agents, coating materials, adhesives, rust preventives, repellents (repellents for human body, repellents for animal), and the like.

<Emulsion Composition for Aerosol Preparation>

The present invention includes an emulsion composition for aerosol preparation, comprising an oil phase; an aqueous phase; and vesicles formed of an amphipathic substance which spontaneously forms the vesicles, or particles of a condensation polymer having hydroxyl groups.

It is possible to use, as the emulsion composition for aerosol preparation of the present invention, emulsion compositions in the above-mentioned aerosol agent.

EXAMPLES

<Preparation 1 of Aerosol Agent>

(Preparation of Starting Solution composed of O/W Three-Phase Emulsion Composition)

[Preparation of Starting Solution composed of Vesicles-containing (B-based) O/W Three-Phase Emulsion Composition]

Dispersions of vesicles of 5 mass% polyoxyethylene hardened castor oil or 5 mass% decaglyceryl distearate were prepared. To each of these dispersions, liquid paraffin and water were added so as to satisfy the formulation shown in Table 1 below, followed by stirring to prepare starting solutions composed of each B-based O/W three-phase emulsion composition.
"B-based stock solution" as used herein means a dispersion of vesicles.

[Table 1]

| Name of raw materials | Mixing amount [g] |
|---|---|
| Liquid paraffin | 10 |

(continued)

| Name of raw materials | Mixing amount [g] |
|---|---|
| B-based stock solution | 5 |
| Water | 85 |
| Total | 100 |

[Preparation of Starting Solution composed of Condensation Polymer Particles-containing (S-based) O/W Three-Phase Emulsion Composition]

From a solution of 0.1 mass% hydroxyethyl cellulose or 0.1 mass% stearoxyhydroxypropylmethyl cellulose, dispersions of each of condensation polymer particles were prepared. To each of these dispersions, liquid paraffin, water, and hydroxyethyl cellulose were added so as to satisfy the formulation shown in Table 2 below, followed by stirring to prepare starting solutions composed of each S-based O/W three-phase emulsion composition.
"S-based stock solution" as used herein means a dispersion of condensation polymer particles.

[Table 2]

| Name of raw materials | Mixing amount [g] |
|---|---|
| Liquid paraffin | 10 |
| S-based stock solution | 10 |
| Water | 79.75 |
| Hydroxyethyl cellulose (For the purpose of thickening dispersion phase) | 0.25 |
| Total | 100 |

(Filling of propellant)

[Filling of Compressed Gas]

Each of the thus prepared starting solution composed of a B-based or S-based O/W three-phase emulsion composition was stored in a pressure-resistant glass bottle, and then the glass bottle was filled with a pressure gas at 25°C so that a pressure of a $N_2$, $CO_2$, or $N_2O$ compressed gas became changeable by 0.2 MPa within a range of 0.2 MPa to 1.0 MPa, relative to 20 g of the starting solution composed of the B-based or S-based O/W three-phase emulsion composition, to prepare respective aerosol agent.

[Filling of Liquefied Gas]

Each of the thus prepared starting solution composed of a B-based or S-based O/W three-phase emulsion composition was stored in a pressure-resistant glass bottle, and then the glass bottle was filled with a butane, propane, dimethyl ether (DME), or 1,3,3,3-tetrafluoroprop-1-ene (1234-ze) liquefied gas (6 g, 15 g, or 24 g) so that the concentration of the liquefied gas became 20% by mass, 50% by mass, or 80% by mass relative to the starting solution (24 g, 15 g, or 6 g) composed of a B-based or S-based O/W three-phase emulsion composition to prepare the respective aerosol agents.

<Evaluation 1>

Regarding each of the aerosol agents prepared in the above-mentioned preparation 1 of the aerosol agent, a compressed gas or a liquefied gas was filled. After 3 days, an emulsified state of the emulsion composition was visually observed and evaluation was performed. The evaluation was performed by the following criteria. G (Good): Free from problem on emulsified state

$B_1$ (Bad): Demulsification partially occurred
$B_2$ (Bad): Demulsification entirely occurred leading to oil-water separation

The results of evaluation on an aerosol agent filled with a compressed gas are shown in Table 3 below, and the results of evaluation on an aerosol agent filled with a liquefied gas are shown in Table 4 below. The symbol "-" in Table 4 means an unmeasured sample. Hereinafter, the same shall apply in tables herein.

[Table 3]

| Compressed gas | | $N_2$ | | $CO_2$ | | $N_2O$ | |
|---|---|---|---|---|---|---|---|
| | | B-based | S-based | B-based | S-based | B-based | S-based |
| Emulsion component | | Polyoxyethylene hardened castor oil | Hydroxyethyl cellulose | Decaglyceryl distearate | Hydroxyethyl cellulose | Polyoxyethylene hardened castor oil | Hydroxyethyl cellulose |
| Pressure of compressed gas [MPa] | 0.2 | G | G | G | G | G | G |
| | 0.4 | G | G | G | G | G | G |
| | 0.6 | G | G | G | G | G | G |
| | 0.8 | G | G | G | G | G | G |
| | 1 | G | G | G | G | G | G |

[Table 4]

| Liquefied gas | | Butane | | | Propane | | | DME | | 1234-ze | | |
|---|---|---|---|---|---|---|---|---|---|---|---|---|
| Emulsion component | | B-based | S-based | | B-based | S-based | | B-based | | B-based | S-based | |
| | | Polyoxy ethylene hardened castor oil | Hydroxyethyl cellulose | Stearoxy hydroxy propylmethyl cellulose | Polyoxy ethylene hardened castor oil | Hydroxyethyl cellulose | Stearoxy hydroxy propylmethyl cellulose | Polyoxy ethylene hardened castor oil | Decaglyceryl distearate | Polyoxy ethylene hardened castor oil | Hydroxyethyl cellulose | Stearoxy hydroxy propylmethyl cellulose |
| Propellant concentration [wt%] | 20 | G | G | G | G | G | G | G | G | G | G | G |
| | 50 | G | G | G | G | G | G | - | G | G | G | G |
| | 80 | G | G | G | G | G | G | - | G | G | G | G |

The results of Table 3 and Table 4 revealed that, regarding an aerosol agent including a starting solution composed of a B-based or S-based O/W three-phase emulsion composition, and a propellant of a compressed gas or a liquefied gas, an emulsified state of the emulsion composition is stable.

<Preparation 2 of Aerosol Agent>

(Preparation of Starting Solution composed of W/O Three-Phase Emulsion Composition)

[Preparation of Starting Solution composed of B-based W/O Three-Phase Emulsion Composition]

Dispersions of vesicles of 5 mass% polyoxyethylene hardened castor oil or 5 mass% decaglyceryl distearate were prepared. To these dispersions, isopropyl myristate was used as the oil during filling with the below-mentioned propellant so as to satisfy the formulation shown in Table 5 below when using a compressed gas, or to satisfy the formulation shown in Table 6 below when using a liquefied gas, followed by stirring to prepare starting solutions composed of each B-based W/O three-phase emulsion composition.

[Table 5]

| Name of raw materials | Mixing amount [g] |
|---|---|
| Isopropyl myristate | 90 |
| B-based stock solution | 10 |
| Total | 100 |

[Table 6]

| Name of raw materials | Mixing amount [g] |
|---|---|
| Isopropyl myristate | 70 |
| B-based stock solution | 30 |
| Total | 100 |

[Preparation of Starting Solution composed of S-based W/O Three-Phase Emulsion Composition]

From a solution of 0.1 mass% agar or 0.1 mass% hydroxyethyl cellulose, dispersions of each of condensation polymer particles were prepared. To these dispersions, diisostearyl malate was used as the oil so as to satisfy the formulation shown in Table 7, followed by stirring to prepare starting solutions composed of each S-based W/O three-phase emulsion composition.

[Table 7]

| Name of raw materials | Mixing amount [g] |
|---|---|
| Diisostearyl malate | 90 |
| S-based stock solution | 10 |
| Total | 100 |

(Filling of Propellant)

[Filling of Compressed Gas]

Using the thus prepared starting solution composed of a B-based or S-based W/O three-phase emulsion composition, filling of a compressed gas was performed in the same manner as in the above-mentioned Preparation 1 of Aerosol Agent to prepare the respective aerosol agents.

[Filling of Liquefied Gas]

In the same manner as in the above-mentioned Preparation 1 of Aerosol Agent, except that water as an outer phase was removed from the starting solution composed of a B-based W/O three-phase emulsion composition in the amount of 70% by mass relative to the total mass of the starting solution, filling of a liquefied gas was performed using the thus prepared starting solution composed of a B-based W/O three-phase emulsion composition to prepare the respective aerosol agents.

<Evaluation 2>

In the same manner as in the above-mentioned Evaluation 1, the aerosol agent prepared in Preparation 2 of Aerosol Agent was visually observed and evaluation was performed.

The results of evaluation on an aerosol agent filled with a compressed gas are shown in Table 8 below, and the results of evaluation on an aerosol agent filled with a liquefied gas are shown in Table 9 below.

[Table 8]

| Compressed gas | | $N_2$ | | | $CO_2$ | | | $N_2O$ | | |
|---|---|---|---|---|---|---|---|---|---|---|
| Emulsion component | | B-based | S-based | | B-based | S-based | | B-based | S-based | |
| | | Polyoxyethylene hardened castor oil | Agar | Hydroxyethyl cellulose | Polyoxyethylene hardened castor oil | Agar | Hydroxyethyl cellulose | Polyoxyethylene hardened castor oil | Agar | Hydroxyethyl cellulose |
| Pressure of compressed gas [MPa] | 0.2 | G | G | G | G | G | G | G | G | G |
| | 0.4 | G | G | G | G | G | G | G | G | G |
| | 0.6 | G | G | G | G | G | G | G | G | G |
| | 0.8 | G | G | G | G | G | G | G | G | G |
| | 1 | G | G | G | G | G | G | G | G | G |

[Table 9]

| Liquefied gas | | Butane | | Propane | DME | 1234-ze |
|---|---|---|---|---|---|---|
| Emulsion component | | B-based | | B-based | B-based | B-based |
| | | Polyoxyethylene hardened castor oil | Decaglyceryl distearate | Polyoxyethylene hardened castor oil | Polyoxyethylene hardened castor oil | Polyoxyethylene hardened castor oil |
| | | Isopropyl myristate | Diisostearyl malate | Isopropyl myristate | Isopropyl myristate | Isopropyl myristate |
| Propellant concentration [wt%] | 20 | G | G | G | G | G |
| | 50 | G | G | G | G | G |
| | 80 | G | G | G | - | G |

The results of Table 8 and Table 9 revealed that, regarding an aerosol agent including a starting solution composed of a B-based or S-based W/O three-phase emulsion composition, and a propellant of a compressed gas or a liquefied gas, an emulsified state of the emulsion composition is stable.

<Preparation 3 of Aerosol Agent>

(Preparation of Starting Solution composed of O/W Emulsion Composition using Surfactant)

Using polyoxyethylene oleyl ether and glyceryl monostearate as surfactants, liquid paraffin as oil, and water so as to satisfy the formulation shown in Table 10 below, starting solutions composed of an O/W emulsion composition using a surfactant were prepared.

[Table 10]

| Name of raw materials | Mixing amount [g] |
|---|---|
| Liquid paraffin | 10 |
| Polyoxyethylene oleyl ether | 0.54 |
| Glyceryl monostearate | 0.46 |
| Water | 89 |
| Total | 100 |

(Preparation of Starting Solution composed of W/O Emulsion Composition using Surfactant)

To prepare a starting solution composed of a W/O emulsion composition using a surfactant according to Formulation 1 or Formulation 2 shown in Table 11 below, polyglyceryl monoisostearate, diglyceryl triisostearate, sorbitan oleate, or polyoxyethylene sorbitan monostearate was used as the surfactant, and diisostearyl malate or liquid paraffin was used as the oil, and also water was used. In case of Formulation 2, dextrin palmitate (Rheopearl KL2, manufactured by Chiba Flour Milling Co., Ltd.) was further used to prepare a starting solution composed of a W/O emulsion composition using a surfactant.

[Table 11]

| Formulation 1 (using surfactant) | | Formulation 2 (using surfactant) | |
|---|---|---|---|
| Name of raw materials | Mixing amount[g] | Name of raw materials | Mixing amount[g] |
| Diisostearyl malate | 80 | Liquid paraffin | 79 |
| Polyglyceryl monoisostearate | 4 | Rheopearl KL2 | 1 |
| Diglyceryl triisostearate | 4 | Sorbitan oleate | 0.1 |
| Water | 12 | Polyoxyethylene sorbitan monostearate | 1.9 |
| Total | 100 | Water | 18 |
| | | Total | 100 |

(Filling of Propellant)

[Filling of Compressed Gas]

Using the thus prepared starting solution composed of an O/W or W/O three-phase emulsion composition, filling of a compressed gas was performed in the same manner as in the above-mentioned Preparation 1 of Aerosol Agent to prepare the respective aerosol agents.

[Filling of Liquefied Gas]

In the same manner as in the above-mentioned Preparation 1 of Aerosol Agent, except that water as an outer

phase was removed from the starting solution composed of a W/O three-phase emulsion composition using a surfactant in the amount of 70% by mass relative to the total mass of the starting solution, filling of a liquefied gas was performed using the thus prepared starting solution composed of an O/W or W/O three-phase emulsion composition to prepare the respective aerosol agents.

<Evaluation 3>

In the same manner as in the above-mentioned Evaluation 1, the aerosol agent prepared in Preparation 3 of Aerosol Agent was visually observed and evaluation was performed.

The results of evaluation on an aerosol agent filled with a compressed gas are shown in Table 12 below, and the results of evaluation on an aerosol agent filled with a liquefied gas are shown in Table 13 below.

[Table 12]

| Compressed gas | | $N_2$ | | $CO_2$ | | $N_2O$ | |
|---|---|---|---|---|---|---|---|
| Emulsion component | | O/W | W/O | O/W | W/O | O/W | W/O |
| | | | Formulation1 | | Formulation1 | | Formulation1 |
| Pressure of compressed gas[MPa] | 0.2 | G | G | G | G | G | G |
| | 0.4 | G | G | G | G | G | G |
| | 0.6 | G | G | G | G | G | G |
| | 0.8 | G | G | G | G | G | G |
| | 1 | G | G | G | G | G | G |

[Table 13]

| Liquefied gas | | Butane | | | Propane | | | DME | | | 1234-ze | |
|---|---|---|---|---|---|---|---|---|---|---|---|---|
| Emulsion component | | O/W | W/O | | O/W | W/O | | O/W | W/O | | O/W | W/O |
| | | | Formulation 1 | Formulation 2 | | Formulation 1 | Formulation 2 | | Formulation 1 | Formulation 2 | | Formulation 2 |
| Propellant concentration [wt%] | 20 | G | G | G | G | G | G | G | $B_2$ | G | G | G |
| | 50 | G | G | G | G | G | G | $B_2$ | $B_2$ | G | G | G |
| | 80 | G | G | G | G | G | G | $B_2$ | $B_2$ | G | G | G |

As shown in Tables 12 and 13, when using dimethyl ether (DME) as the liquefied gas, an emulsified state of the emulsion composition was not sometimes stable in the aerosol agent using either an O/W or W/O emulsion composition. Whereas, in the aerosol agent using a three-phase emulsion composition, when using dimethyl ether (DME) as the liquefied gas, an emulsified state of the emulsion composition was stable as shown in Table 4 and Table 9 above.

<Preparation 4 of Aerosol Agent>

(Preparation of Starting Solution composed of W/O Three-Phase Emulsion Composition)

[Preparation of Starting Solution composed of B-based W/O Three-Phase Emulsion Composition]

Dispersions of vesicles of 5 mass% polyoxyethylene hardened castor oil were prepared. To these dispersions, liquid paraffin containing dextrin palmitate (Rheopearl KL2, manufactured by Chiba Flour Milling Co., Ltd.) was added so as to satisfy the formulation shown in Table 14 below, followed by stirring to prepare starting solutions composed of a B-based W/O three-phase emulsion composition.

[Preparation of Starting Solution composed of S-based W/O Three-Phase Emulsion Composition]

From a solution of 0.1 mass% hydroxyethyl cellulose, dispersions of condensation polymer particles were prepared. To these dispersions, liquid paraffin containing dextrin palmitate (Rheopearl KL2, manufactured by Chiba Flour Milling Co., Ltd.) was added so as to satisfy the formulation as shown in Table 14, followed by stirring to prepare starting solutions composed of an S-based W/O three-phase emulsion composition.

[Table 14]

| Name of raw materials | Mixing amount [g] |
|---|---|
| Liquid paraffin | 89.1 |
| B-based stock solution or S-based stock solution | 10 |
| Rheopearl KL2 | 0.9 |
| Total | 100 |

(Filling of Propellant)

[Filling of Liquefied Gas]

In the same manner as in the above-mentioned Preparation 1 of Aerosol Agent, except that water as an outer phase was removed from the starting solution composed of a B-based or S-based W/O three-phase emulsion composition in the amount of 70% by mass relative to the total mass of the starting solution, filling of a liquefied gas was performed using the thus prepared starting solution composed of a B-based or S-based W/O three-phase emulsion composition to prepare the respective aerosol agents.

<Evaluation 4>

In the same manner as in the above-mentioned Evaluation 1, the aerosol agent prepared in Preparation 4 of Aerosol Agent was visually observed and evaluation was performed.

The results of evaluation on an aerosol agent filled with a liquefied gas are shown in Table 15 below.

[Table 15]

| Liquefied gas | | Butane | | Propane | | DME | | 1234-7e | |
|---|---|---|---|---|---|---|---|---|---|
| Emulsion component | | Polyoxyethylene hardened castor oil | Hydroxyethyl cellulose | Polyoxyethylene hardened castor oil | Hydroxyethyl cellulose | Polyoxyethylene hardened castor oil | Hydroxyethyl cellulose | Polyoxyethylene hardened castor oil | Hydroxyethyl cellulose |
| Propellant concentration [wt%] | 20 | G | G | G | G | G | G | G | G |
| | 50 | G | G | G | G | G | G | G | G |
| | 80 | G | G | G | G | - | - | G | G |

The results of Table 15 revealed that, when dextrin palmitate is added to an oil phase in a starting solution composed of B-based or S-based W/O three-phase emulsion composition, an emulsified state of the emulsion composition containing liquid paraffin becomes stable.

Using water in place of the B-based stock solution or the S-based stock solution in Table 14 above, a composition was prepared. This composition and the S-based W/O three-phase emulsion composition were observed by a microscope. The results are shown in Fig. 1. In Fig. 1, Fig. 1A shows an image of an S-based W/O three-phase emulsion composition, which is observed by a microscope, and Fig. 1B shows an image of a composition containing neither a B-based stock solution nor an S-based stock solution prepared by using water in place of the B-based stock solution or the S-based stock solution in Table 14, which is observed by a microscope. As shown in Fig. 1, it has been confirmed the S-based W/O three-phase emulsion composition is emulsified, whereas, the composition containing neither a B-based stock solution nor an S-based stock solution is not emulsified.

<Preparation 5 of Aerosol Agent>

(Preparation of Starting Solution composed of O/W Three-Phase Emulsion Composition)

[Preparation of Starting Solution composed of S-based O/W Three-Phase Emulsion Composition]

A solution of 0.1 mass% hydroxyethyl cellulose was converted into dispersions of condensation polymer particles. To these dispersions, liquid paraffin containing an acrylate copolymer (Carbopol Aqua SF-1 OS polymer, manufactured by Lubrizol Advanced Materials, Inc.) was added so as to satisfy the formulation shown in Table 16 below, followed by stirring to prepare starting solutions composed of an S-based O/W three-phase emulsion composition. "Carbopol Aqua SF-1 OS polymer" is a copolymer composed of two or more monomers of alkyl acrylate, alkyl methacrylate, acrylic acid, or methacrylic acid.

[Table 16]

| Name of raw materials | Mixing amount [g] |
|---|---|
| Liquid paraffin | 10 |
| B-based stock solution or S-based stock solution | 5 |
| 3 % by mass Carbopol Aqua SF-1 OS polymer | 85 |
| Total | 100 |

(Filling of Propellant)

[Filling of Liquefied Gas]

Using the thus prepared starting solution composed of an S-based O/W three-phase emulsion composition, filling of a liquefied gas was performed in the same manner as in the above-mentioned Preparation 1 of Aerosol Agent to prepare the respective aerosol agents.

<Evaluation 5>

In the same manner as in the above-mentioned Evaluation 1, the aerosol agent prepared in Preparation 5 of Aerosol Agent was visually observed and evaluation was performed.

The results of evaluation on an aerosol agent filled with a liquefied gas are shown in Table 17 below. "Additive" in Table 17 means "acrylate copolymer".

[Table 17]

| Liquefied gas | | DME |
|---|---|---|
| Emulsion component in Table 16 | | Hydroxyethyl cellulose+ additives |
| Propellant concentration [wt%] | 20 | G |
| | 50 | G |
| | 80 | G |

The results of Table 17 revealed that, when an acrylate copolymer is added to an oil phase in a starting solution composed of an S-based O/W three-phase emulsion composition, an emulsified state of the emulsion composition is stable.

<Preparation 6 of Aerosol Agent>

(Preparation of Starting Solution composed of O/W Three-Phase Emulsion Composition)

[Preparation of Starting Solution composed of S-based O/W Three-Phase Emulsion Composition]

From a solution of 0.1 mass% hydroxyethyl cellulose, dispersions of condensation polymer particles were prepared. To these dispersions, liquid paraffin and water were added so as to satisfy the formulation shown in Table 18 below, followed by stirring to prepare starting solutions composed of an S-based O/W three-phase emulsion composition.

[Table 18]

| Name of component | Mixing amount [g] |
|---|---|
| Liquid paraffin | 10 |
| Water | 70 |
| 0.1% Hydroxyethyl cellulose stock solution | 20 |

(Filling of Propellant)

[Filling of Compressed Gas]

The thus prepared starting solution composed of an S-based O/W three-phase emulsion composition was stored in a pressure-resistant glass bottle, and then the glass bottle was filled with a $N_2$ compressed gas so that the pressure became 0.8 MPa at 25°C relative to 30 g of the starting solution composed of an S-based O/W three-phase emulsion composition to prepare aerosol agents according to preparation 6.

<Preparation 7 of Aerosol Agent>

(Preparation of Starting Solution composed of O/W Emulsion Composition using Surfactant)

Using polyoxyethylene oleyl ether and glyceryl monostearate as surfactants, liquid paraffin as oil, and water so as to satisfy the formulation shown in Table 19 below, starting solutions composed of an O/W emulsion composition using a surfactant were prepared.

[Table 19]

| Name of component | Mixing amount [g] |
|---|---|
| Liquid paraffin | 10 |
| Water | 90 |
| Polyoxyethylene oleyl ether | 0.54 |
| Glyceryl monostearate | 0.46 |

(Filling of Propellant)

[Filling of Compressed Gas]

Using the thus prepared starting solution composed of an O/W emulsion composition, filling of a compressed gas was performed in the same manner as in Preparation 6 of Aerosol Agent, aerosol agents according to Preparation 7 were prepared.

<Evaluation 6>

Using the aerosol agent according to preparation 6 and the aerosol agents according to preparation 7, adhesion to a solid surface was evaluated.

The measurement was performed by the following procedure.

(Preparation of Measuring Plate)

First, a filter paper (QUALITATIVE ITEM1, 600 mm × 600 mm) was cut into quarters each having a size of 300 mm × 300 mm. Next, the filter paper was fixed to an aluminum plate erected vertically using a clip. After fixation, the aluminum plate was placed on a balance and zero-point adjustment was performed.

(Measurement of Adhesion Ratio)

First, each aerosol agent was immersed in a constant temperature water bath at 15°C for 30 minutes or more in a state of being filled in a container. After permeation, the aerosol agent was taken out from the constant temperature water bath. After sufficiently wiping off moisture, an actuator was mounted on the container filled with the aerosol agent, followed by weighing, and this value was set to "W1". Then, in an atmosphere not to be affected by an air current, each aerosol agent was fixed at a distance of 15 cm or 20 cm from the measuring plate. After spraying for 5 seconds, the contents adhered to the filter paper were weighed. The value of the balance is an adhesion amount of the contents, and this value was set to "W2". The weight of the aerosol agent after spraying was measured in a state where the actuator is mounted on the container, and this value was set to "W3". Using these values W1 to W3, an adhesion ratio was calculated by the calculation formula mentioned below. N=5 in case of spraying from a distance of 15 cm, and N=3 in case of spraying from a distance of 20 cm.

$$\text{Adhesion ratio (\%)} = W2/(W1 - W3) \times 100$$

Regarding spraying at a distance of 15 cm from the measuring plate, the adhesion ratio of the aerosol agent according to preparation 6 (aerosol agent according to three-phase emulsification) was 55.70%, whereas, the adhesion ratio of the aerosol agent according to preparation 7 (aerosol agent according to surfactant emulsification) was 46.68%. Regarding spraying at a distance of 20 cm from the measuring plate, the adhesion ratio of the aerosol agent according to preparation 6 was 50.44%, whereas, the adhesion ratio of the aerosol agent according to preparation 7 (aerosol agent according to surfactant emulsification) was 26.61%.

An average particle diameter of sprayed particles of each aerosol agent was measured under the conditions of the number of measurements N=3. As a result, the average particle diameter of sprayed particles of the aerosol agent according to preparation 6 was 44.95 μm, whereas, the average particle diameter of sprayed particles of the aerosol agent according to preparation 7 was 43.52, thus confirming that there is little difference. The average particle diameter of sprayed particles was measured by a LDSA particle size analyzer (LDSA-3400A: NIKKISO CO., LTD.).

The results revealed that, when the three-phase emulsion composition is used as the starting solution of an aerosol agent, the adhesion ratio is higher than that when using the emulsion composition using a surfactant. The results also revealed the followings. If the distance from the object increases, the adhesion ratio significantly decreases in the aerosol agent using an emulsion composition using a surfactant, whereas, the adhesion ratio is less likely to decrease in the aerosol agent using a three-phase emulsion composition. In other words, as the distance from the object increases, adhesion of the aerosol agent using a three-phase emulsion composition becomes noticeably excellent.

## Claims

1. An emulsion composition for an aerosol preparation comprising an oil phase; an aqueous phase; and vesicles formed of an amphipathic substance which spontaneously forms the vesicles, or particles of a condensation polymer having hydroxyl groups the oil phase including a polymer compound having hydrophilic groups and hydrophobic groups, and the vesicles or the particles of a condensation polymer existing at an interface between the oil phase and the aqueous phase.

2. An aerosol agent comprising a starting solution composed of an emulsion composition, and a propellant, the emulsion composition including an oil phase; an aqueous phase; and vesicles formed of an amphipathic substance which

spontaneously forms the vesicles, or particles of a condensation polymer having hydroxyl groups, the oil phase including a polymer compound having hydrophilic groups and hydrophobic groups, and the vesicles or the particles of a condensation polymer existing at an interface between the oil phase and the aqueous phase.

**3.** The aerosol agent according to claim 2 for application to a solid surface.

**Patentansprüche**

**1.** Emulsionszusammensetzung für ein Aerosolpräparat, umfassend eine Ölphase; eine wässerige Phase; und Vesikel, die aus einer amphipathischen Substanz gebildet sind, die spontan die Vesikel bildet, oder Teilchen eines Kondensationspolymers mit Hydroxylgruppen, wobei die Ölphase eine Polymerverbindung mit hydrophilen Gruppen und hydrophoben Gruppen umfasst und die Vesikel oder die Teilchen eines Kondensationspolymers an einer Grenzfläche zwischen der Ölphase und der wässerigen Phase vorhanden sind.

**2.** Aerosolmittel, umfassend eine Ausgangslösung, die aus einer Emulsionszusammensetzung und einem Treibmittel zusammengesetzt ist, wobei die Emulsionszusammensetzung eine Ölphase; eine wässerige Phase; und Vesikel, die aus einer amphipathischen Substanz gebildet sind, die spontan die Vesikel bildet, oder Teilchen eines Kondensationspolymers mit Hydroxylgruppen umfasst, wobei die Ölphase eine Polymerverbindung mit hydrophilen Gruppen und hydrophoben Gruppen umfasst und die Vesikel oder die Teilchen eines Kondensationspolymers an einer Grenzfläche zwischen der Ölphase und der wässerigen Phase vorhanden sind.

**3.** Aerosolmittel nach Anspruch 2 zur Anwendung auf eine feste Oberfläche.

**Revendications**

**1.** Composition d'émulsion pour une préparation d'aérosol comprenant une phase huileuse ; une phase aqueuse ; et des vésicules formées d'une substance amphipathique qui forme spontanément les vésicules, ou particules d'un polymère de condensation ayant des groupes hydroxyle, la phase huileuse comportant un composé polymère ayant des groupes hydrophiles et des groupes hydrophobes, et les vésicules ou les particules d'un polymère de condensation existant au niveau d'une interface entre la phase huileuse et la phase aqueuse.

**2.** Agent aérosol comprenant une solution de départ composée d'une composition d'émulsion, et d'un propulsif, la composition d'émulsion comprenant une phase huileuse ; une phase aqueuse ; et des vésicules formées d'une substance amphipathique qui forme spontanément les vésicules, ou particules d'un polymère de condensation ayant des groupes hydroxyle, la phase huileuse comprenant un composé polymère ayant des groupes hydrophiles et des groupes hydrophobes, et les vésicules ou les particules d'un polymère de condensation existant au niveau d'une interface entre la phase huileuse et la phase aqueuse.

**3.** Agent aérosol selon la revendication 2 pour son application sur une surface solide.

FIG. 1A

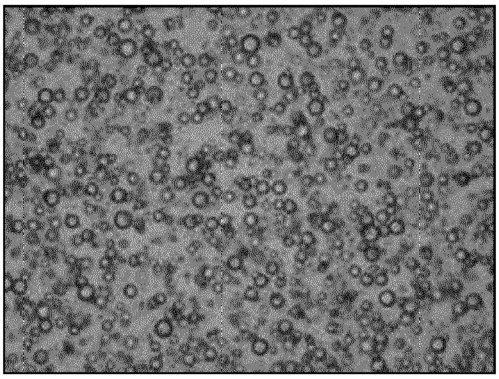

FIG. 1B

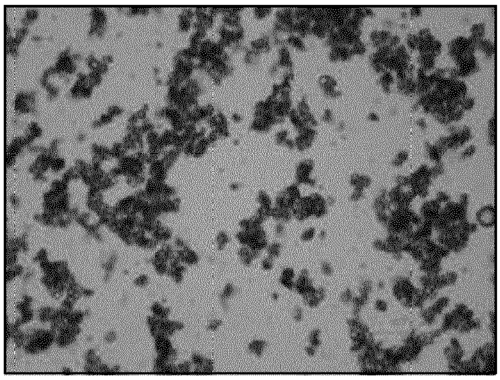

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3399578 B
- JP 3855203 B